# EUROPEAN PATENT SPECIFICATION

(11) **EP 2 611 819 B1**
(45) Date of publication and mention of the grant of the patent: **16.12.2015**
(21) Application number: 11757961.5
(22) Date of filing: 30.08.2011
(51) Int. Cl.: C07F 15/00

(54) **IRIDIUM COMPLEX AND METHOD FOR PRODUCING OPTICALLY ACTIVE COMPOUND**
IRIDIUMKOMPLEX UND VERFAHREN ZUR HERSTELLUNG OPTISCH AKTIVER VERBINDUNGEN
COMPLEXE D'IRIDIUM ET PROCÉDÉ POUR PRODUIRE UN COMPOSÉ OPTIQUEMENT ACTIF

(30) Priority: 30.08.2010 JP 2010192712
(43) Date of publication of application: 10.07.2013
(73) Proprietor: Takasago International Corporation, Tokyo 144-8721 (JP)
(72) Inventor: SHIMIZU, Hideo, Tokyo 144-8721 (JP); NARA, Hideki, Hiratsuka-shi Kanagawa 254-0073 (JP)
(74) Representative: Zimmermann & Partner Patentanwälte mbB
(86) International application number: PCT/JP2011/070092
(87) International publication number: WO 2012/029970

(56) References cited:
- LUTZ DAHLENBURG ET AL: "Synthesis and Catalytic Applications of Chiral Hydridoiridium(III) Complexes with Diamine/Bis(monophosphane) and Diamine/Diphosphane Coordination", EUROPEAN JOURNAL OF INORGANIC CHEMISTRY, vol. 2007, no. 27, 1 September 2007 (2007-09-01), pages 4364-4374, XP55011954, ISSN: 1434-1948, DOI: 10.1002/ejic.200700380
- ROMANO DORTA ET AL: "Facile and Reversible O-H and C-H Activation by a Chiral Iridium(I) Complex", ORGANOMETALLICS, vol. 17, no. 16, 1 August 1998 (1998-08-01), pages 3423-3428, XP55011961, ISSN: 0276-7333, DOI: 10.1021/om980189b

## Description

### Technical Field

The present invention relates to an iridium complex and a method for producing an optically active compound.

### Background Art

Synthetic methods based on catalytic asymmetric hydrogenation reactions are still being actively investigated as methods for synthesizing optically active compounds. Complexes containing rhodium, ruthenium, or iridium are mainly used as catalysts for asymmetric hydrogenation reactions. Among these complexes, complexes containing iridium can be applied to different varieties of substrates from those of rhodium and ruthenium, and hence are catalysts useful for synthesis.

Addition of an additive to an iridium complex often leads to improvement in the activity and selectivity of the catalyst. Hence, various additives have been proposed. For example, iodine in Angew. Chem. Int. Ed. Engl. 1996, 35, 1475, tetrabutylammonium bromide in Chem. Pharm. Bull. 1994, 42, 1951, bismuth iodide in Synlett 1995, 748, phthalimide in Tetrahedron: Asymmetry 1998, 9, 2415, piperidine hydrochloride in Adv. Synth. Catal. 2009, 351, 2549, and protic amines such as benzylamine in Chem. Lett. 1995, 955 have been reported as effective additives for improving the activity and selectivity of catalysts. However, there has been a problem that, for some substrates, sufficient catalytic activities and enantiomeric excesses cannot be achieved even with these additives.
L. Dahlenburg et al., "Synthesis and Catalytic Applications of Chiral Hydridoiridium(III) Complexes with Diamine/Bis(monophosphane) and Diamine/Diphosphane Coordintion", Eur. J. Inorg. Chem. 2007, 4364-4374 describe that the P₂/N₂-coordinated cis-dihydrridoiridium(III) chelate complexes (OC-6-13) - [IrH₂(H₂N∩NH₂)(PR₃)₂] BF₄ [PR₃ = PPh₃, H₂N∩NH₂ = 1,2-(H₂N)₂C₆H₄ (1a) ; (1*R*, 2*R*) *-* (H₂N) ₂ C₆H₁₀ {(-*R*,-*R*)-dach} (1b); (*R*)-2,2'-diamino-1,1'-binaphthyl {(*R*)-dabin} (1c) ; PR₃ = PiPr₃, H₂N∩NH₂ = (*R,R*)-dach (2a), (*R*)-dabin (2b); PR₃ = PCy₃, H₂N∩NH₂ = (*R*)-dabin (3)] were obtained by treating the respective diamine ligands with labile precursors such as [IrH₂(OCMe₂)₂(PPh₃)₂]BF₄, [(η⁴-1,5-C₈H₁₂)Ir(PPH₃)₂]BF₄, or [IrH₅(PR₃)₂]/HBF₄ (R = iPr, Cy). While oxidative addition of HCl to [(η⁴-1,5-C₈H₁₂)Ir{(*S*,*S*)-bdpcp}]BF₄
[(S,S)-bdpcp = (1*S*,2*S*)-(Ph₂P)₂C₅H₈] yields the usual mononuclear adduct [(η⁴-1,5-C₈H₁₂)Ir(H)(Cl){(*S*,*S*)-bdpcp}]BF₄ (4), similar treatment of [(η⁴-1,5-C₈H₁₂)Ir{(R) -binap}] furnishes the triply chloride-bridged diiridium complex [{(R)-binap}₂]Ir₂H₂(µ-Cl)₃] (5). Opening of the µ-bridges of 5 by N,N nucleophiles was used to synthesize the three diamine/(R)-binap complexes [Ir(H)(Cl)-(H₂NnNH₂){(R)-binap}]BF₄ [H₂N∩NH₂ = (1*R*,2*R*)-H₂NCH(Ph)NH₂ {(*R*,-*R*)-dpen} (6a), (R,R)-dach (6b), and H₂NCMe₂CMe₂NH₂ {tmen} (6c). Whereas the dihydrides [IrH₂{(R,R)-dach} (PR₃)₂]BF₄ and [IrH₂{(R)-dabin}-(PR₃)₂]BF₄ (R = *i*Pr, Ph) are only poor (pre)-catalysts for the enantioselective hydrogenation of acetophenone, complexes 6a-6c catalyze the formation of 1-phenyletahnol in good enantiomeric excess [eeₘₐₓ = 82-84 % (*S*)] in the presence of base. The crystal structures of 1a, 4 and 5 have been determined.
More specifically, Dahlenburg et al. have searched for complexes of the Group 9 transition metals with possibly improved catalytic properties, and they have synthesized and investigated some cationic hydrido compounds of iridium(III) with coordination spheres dominated by chiral (di)phosphane ligands, namely [IrH₂(H₂N∩NH₂)(PR₃)₂]BF₄ (R = Ph, iPr, Cy) and [Ir(H)(X)(H₂N∩NH₂)(Ph₂P∩PPh₂)]BF₄ (X + H or Cl).
R. Dorta and A. Togni, "Facile and reversible O-H and C-H Activation by a Chiral Iridium(I) Complex", Organometallics 1998, 17, 3423-3428 disclose that the reactivity of the dinuclear Ir(I) complexes *cis-* and *trans-* [Ir₂(µ-Cl)2((*R*)-(*S*)-PPFPPh₂)₂] (1, (*R*)-(*S*)-PPFPPH₂ = (-*R*)-1-({(S)-2-(diphenylphosphino)fe rrocenyl}ethyldiphenylphosphine) toward O-H and C-H bonds has been studied (1 was previously shown to be a catalyst precursor for the asymmetric addition of aniline to norbornene, via N-H activation). Compound 1 undergoes clean oxidative addition of water in toluene, affording a mixture of two isomeric, dinuclear hydroxo-bridged Ir(III) complexes. Isomerization to a single product, *syn-trans-*[((*R*)-(*S*)-PPFPPh₂)₂Ir₂(H)₂(µ-OH)₂] (3), takes place upon dissolution in THF. *syn-trans*-3 has been characterized by X-ray diffraction. Reaction of 1 with 2,6-dimethylaniline affords the corresponding sp³ C-H activation product 5, the crystal structure of which has been determined. Both oxidative addition reactions are (partly) reversible at high temperatures.

### Summary of Invention

An object of the present invention is to provide a novel iridium complex, and to provide a novel catalyst having excellent performances in terms of enantioselectivity, catalytic activity, and the like. Moreover, the present invention exhibits excellent performances as a catalyst for asymmetric synthesis reaction, in particular, for asymmetric hydrogenation reaction.

The present inventors have made keen examination to develop a novel iridium complex and an asymmetric synthesis reaction using the iridium complex. As a result, the present inventors have found the iridium complex represented by the general formula (1), and have found that asymmetric induction can be achieved in a reaction using the iridium complex. These findings have led to the completion of the present invention. Specifically, the present invention includes the following contents [1] to [7].
[1] An iridium complex of the following general formula (1) :

   IrHZ₂(PP)(Q)ₘ (1)

   wherein Z represents a halogen atom selected from the group consisting of a chlorine atom, a bromine atom and a iodine atom, PP represents a bisphosphine, Q represents an amine, and m represents 1 or 2, wherein
   PP in the general formula (1) is an optically active bisphosphine, wherein
   Q in the general formula (1) is an amine represented by the following general formula (3): 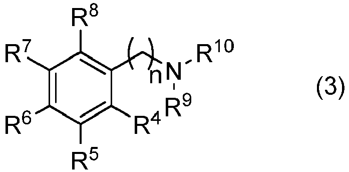 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1, and wherein
      the optically active bisphosphine of the general formula (1) is an optically active bisphosphine represented by the following formula (4) or (5): 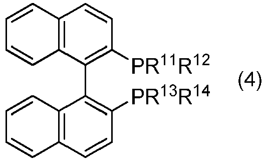 wherein R¹¹, R¹² , R¹³ , and R¹⁴ each independently represent a phenyl group which may have a substituent selected from alkyl groups and alkoxy groups, 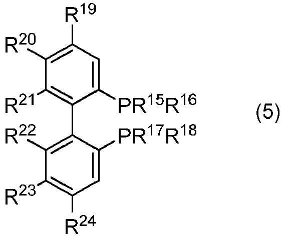 wherein R¹⁵, R¹⁶, R¹⁷, and R¹⁸ each independently represent a phenyl group which may have a substituent selected from alkyl groups and alkoxy groups; R¹⁹, R²⁰, R²¹, R²², R²³, and R²⁴ may be the same or different, and each represent a hydrogen atom, an alkyl group, an alkoxy group, an acyloxy group, a halogen atom, a haloalkyl group, or a dialkylamino group; R²⁰ and R²¹, as well as R²² and R²³, may form a methylene chain which may have a substituent or a (poly)methylenedioxy group which may have a substituent; and R²¹ and R²² may form a methylene chain which may have a substituent or a (poly)methylenedioxy group which may have a substituent, provided that neither R²¹ and R²² are a hydrogen atom.
[2] A method for producing an iridium complex of the following general formula (1*):

   IrHZ₂ (PP*) (Q)ₘ (1*)

   wherein Z represents a halogen atom, PP* represents an optically active bisphosphine, Q represents an amine, and m represents 1 or 2,
   the method comprising allowing one or more equivalents of an amine or a salt thereof to react with an iridium complex represented by the following general formula (6):

      [{IrH(PP*)}₂(µ-Z)₃]Z (6)

      wherein Z represents a halogen atom, and PP* represents an optically active bisphosphine.
[3] A method for producing an iridium complex of the following general formula (1*):

   IrHZ₂ (PP*) (Q)ₘ (1*)

   wherein Z represents a halogen atom, PP* represents an optically active bisphosphine, Q represents an amine, and m represents 1 or 2,
   the method comprising allowing one or more equivalents of an amine or a salt thereof to react with an iridium complex represented by the following general formula (7), and subsequently allowing one or more equivalents of a hydrogen halide HZ (where Z represents a halogen atom) or an aqueous solution thereof to react therewith:

      [IrZ(PP*)]₂ (7)

      wherein Z represents a halogen atom, and PP* represents an optically active bisphosphine.
[4] The production method according to [2] or [3], wherein
   the amine is an amine represented by the following general formula (2):

   NR¹R²R³ (2)

   wherein R¹, R² and R³ each independently represent a hydrogen atom, an alkyl group having 1 to 6 carbon atoms, or an aryl group which may have a substituent.
[5] The production method according to [2] or [3], wherein
   the amine is an amine represented by the following general formula (3): 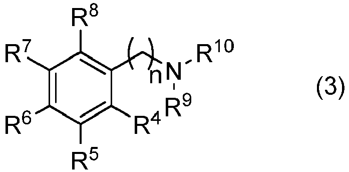 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1.
[6] An asymmetric hydrogenation catalyst comprising the iridium complex according to [1].
[7] A method for producing an optically active compound, comprising performing an asymmetric hydrogenation of a compound having a prochiral carbon-carbon double bond, a prochiral carbon-oxygen double bond and/or a prochiral carbon-nitrogen double bond, or a (hetero)aromatic compound, wherein
   the asymmetric hydrogenation is performed in the presence of an iridium complex represented by the following general formula (6):

   [{IrH(PP*)}₂(µ-Z)₃]Z (6)

   wherein Z represents a halogen atom and PP* represents an optically active bisphosphine, and in the presence of an amine represented by the following general formula (3) or a salt thereof: 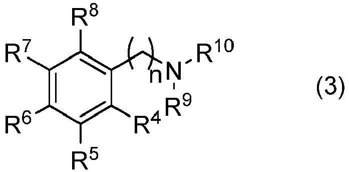 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1.

The use, as a catalyst, of the iridium complex specified in the present invention makes it possible to perform reactions in high yields or in highly stereoselective manners, and to obtain optically active compounds. These optically active compounds are useful as synthetic intermediates for various compounds.

### Description of Embodiments

Hereinafter, the present invention will be described.

As represented by the general formula (1), an iridium complex of the present invention is a complex which contains an iridium atom, halogen atoms, a hydrogen atom, an amine, and a bisphosphine.

The halogen atom represented by Z in the general formula (1) is a chlorine atom, a bromine atom, or an iodine atom.

The bisphosphine represented in the above-mentioned formula (1) by PP is an optically active bisphosphine represented by the following formula (4) or (5) : 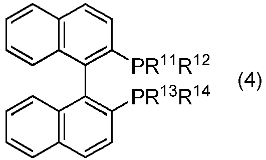 wherein R¹¹, R¹², R¹³, and R¹⁴ each independently represent a phenyl group which may have a substituent selected from alkyl groups and alkoxy groups, 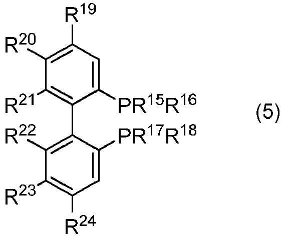 wherein R¹⁵, R¹⁶, R¹⁷, and R¹⁸ each independently represent a phenyl group which may have a substituent selected from alkyl groups and alkoxy groups; R¹⁹, R²⁰, R²¹, R²², R²³, and R²⁴ may be the same or different, and each represent a hydrogen atom, an alkyl group, an alkoxy group, an acyloxy group, a halogen atom, a haloalkyl group, or a dialkylamino group; R²⁰ and R²¹, as well as R²² and R²³, may form a methylene chain which may have a substituent or a (poly)methylenedioxy group which may have a substituent; and R²¹ and R²² may form a methylene chain which may have a substituent or a (poly)methylenedioxy group which may have a substituent, provided that neither R²¹ and R²² are a hydrogen atom.

Examples of the optically active bisphosphine include optically active bisphosphines known before the filing of the present application, and one example thereof is a bisphosphine represented by the general formula (4): 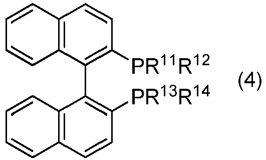 wherein R¹¹, R¹², R¹³ and R¹⁴ each independently represent a phenyl group which may have a substituent selected from alkyl groups and alkoxy groups.

Specific examples of the bisphosphine represented by the general formula (4) include 2,2'-bis-(diphenylphosphino)-1,1'-binaphthyl (hereinafter referred to as BINAP), 2,2'-bis-(di-p-tolylphosphino)-1,1'-binaphthyl (hereinafter referred to as Tol-BINAP), 2,2'-bis-(di-m-tolylphosphino)-1,1'-binaphthyl, 2,2'-bis(di-3,5-xylylphosphino)-1,1'-binaphthyl (hereinafter referred to as DM-BINAP), 2,2'-bis(di-p-tertiary-butylphenylphosphino)-1,1'-bi naphthyl, 2,2'-bis(di-p-methoxyphenylphosphino)-1,1'-binaphthy 1, 2,2'-bis(di-p-chlorophenylphosphino)-1,1'-binaphthyl, 2,2'-bis(dicyclopentylphosphino)-1,1'-binaphthyl (Cp-BINAP), 2,2'-bis(dicyclohexylphosphino)-1,1'-binaphthyl (Cy-BINAP), and the like.

Moreover, another example of the optically active bisphosphine used in the present invention is a bisphosphine represented by the following general formula (5) : 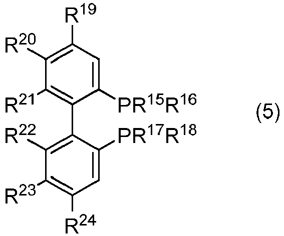 wherein R¹⁵, R¹⁶, R¹⁷, and R¹⁸ each independently represent a phenyl group which may have a substituent selected from alkyl groups and alkoxy groups; R¹⁹, R²⁰, R²¹, R²², R²³ and R²⁴ may be the same or different, and each represent a hydrogen atom, an alkyl group, an alkoxy group, an acyloxy group, a halogen atom, a haloalkyl group, or a dialkylamino group; R²⁰ and R²¹, as well as R²² and R²³, may form a methylene chain which may have a substituent or a (poly)methylenedioxy group which may have a substituent; and R²¹ and R²² may form a methylene chain which may have a substituent or a (poly)methylenedioxy group which may have a substituent, provided that neither R²¹ and R²² are a hydrogen atom.

Specific examples of the bisphosphine represented by the general formula (5) include bisphosphines such as
2,2'-bis(diphenylphosphino)-5,5',6,6',7,7',8,8'-octa hydro-1,1'-binaphthyl (hereinafter referred to as H₈-BINAP),
2,2'-bis(di-p-tolylphosphino)-5,5',6,6',7,7',8,8'-oc tahydro-1,1'-binaphthyl,
2,2'-bis(di-m-tolylphosphino)-5,5',6,6',7,7',8,8'-oc tahydro-1,1'-binaphthyl,
2,2'-bis(di-3,5-xylylphosphino)-5,5',6,6',7,7',8,8'-octahydro-1,1'-binaphthyl,
2,2'-bis(di-p-tertiary-butylphenylphosphino)-5,5',6, 6',7,7',8,8'-octahydro-1,1'-binaphthyl,
2,2'-bis(di-p-methoxyphenylphosphino)-5,5',6,6',7,7' ,8,8'-octahydro-1,1'-binaphthyl,
2,2'-bis(di-p-chlorophenylphosphino)-5,5',6,6',7,7', 8,8'-octahydro-1,1'-binaphthyl,
2,2'-bis(dicyclopentylphosphino)-5,5',6,6',7,7',8,8' -octahydro-1,1'-binaphthyl,
2,2'-bis(dicyclohexylphosphino)-5,5',6,6',7,7',8,8'-octahydro-1,1'-binaphthyl,
((4,4'-bi-1,3-benzodioxole)-5,5'-diyl)bis(diphenylph osphine) (SEGPHOS),
(4,4'-bi-1,3-benzodioxole)-5,5'-diyl)bis(bis(3,5-xyl yl)phosphine) (DM-SEGPHOS),
((4,4'-bi-1,3-benzodioxole)-5,5'-diyl)bis(bis(3,5-di -t-butyl-4-methoxyphenyl)phosphine) (DTBM-SEGPHOS),
((4,4'-bi-1,3-benzodioxole)-5,5'-diyl)bis(bis(4-meth oxyphenyl)phosphine),
((4,4'-bi-1,3-benzodioxole)-5,5'-diyl)bis(dicyclohex ylphosphine) (Cy-SEGPHOS),
((4,4'-bi-1,3-benzodioxole)-5,5'-diyl)bis(bis(3,5-di -t-butylphenyl)phosphine),
2,2'-bis(diphenylphosphino)-4,4',6,6'-tetramethyl-5, 5'-dimethoxy-1,1'-biphenyl,
2,2'-bis(di-p-methoxyphenylphosphino)-4,4',6,6'-tetr amethyl-5,5'-dimethoxy-1,1'-biphenyl,
2,2'-bis(diphenylphosphino)-4,4',6,6'-tetra(trifluor omethyl)-5,5'-dimethyl-1,1'-biphenyl,
2,2'-bis(diphenylphosphino)-4,6-di(trifluoromethyl)-4',6'-dimethyl-5'-methoxy-1,1'-biphenyl,
2-dicyclohexylphosphino-2'-diphenylphosphino-4,4',6, 6'-tetramethyl-5,5'-dimethoxy-1,1'-biphenyl,
2,2'-bis(diphenylphosphino)-6,6'-dimethyl-1,1-biphen yl,
2,2'-bis(diphenylphosphino)-4,4',6,6'-tetramethyl-1, 1'-biphenyl,
2,2'-bis(diphenylphosphino)-3,3',6,6'-tetramethyl-1, 1'-biphenyl),
2,2'-bis(diphenylphosphino)-4,4'-difluoro-6,6'-dimet hyl-1,1'-biphenyl,
2,2'-bis(diphenylphosphino)-4,4'-bis(dimethylamino)-6,6'-dimethyl-1,1'-biphenyl,
2,2'-bis(di-p-tolylphosphino)-6,6'-dimethyl-1,1'-bip henyl,
2,2'-bis(di-o-tolylphosphino)-6,6'-dimethyl-1,1'-bip henyl,
2,2'-bis(di-m-fluorophenylphosphino)-6,6'-dimethyl-1 ,1'-biphenyl,
1,11-bis(diphenylphosphino)-5,7-dihydrobenzo[c,e]oxe pin,
2,2'-bis(diphenylphosphino)-6,6'-dimethoxy-1,1'-biph enyl,
2,2'-bis(diphenylphosphino)-5,5',6,6'-tetramethoxy-1 ,1'-biphenyl,
2,2'-bis(di-p-tolylphosphino)-6,6'-dimethoxy-1,1'-bi phenyl,
2,2'-bis(diphenylphosphino)-4,4',5,5',6,6'-hexametho xy-1,1'-biphenyl, and the like.

The amine represented by Q is an amine represented by the following general formula (3): 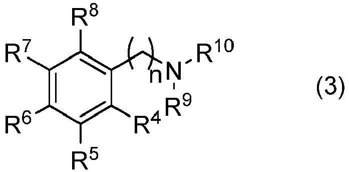 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1.

Specific examples of the amine include aniline, N-methylaniline, N,N-dimethylaniline, toluidine, N-methyltoluidine, N,N-dimethyltoluidine, p-anisidine, N-methyl-p-anisidine, N,N-dimethyl-p-anisidine, benzylamine, N-methylbenzylamine, 1-phenylethylamine, and the like.

Examples of the halogen atom and the optically active bisphosphine in the iridium complex represented by the general formula (6) or (7), which is a synthetic precursor of the iridium complex represented by the general formula (1) of the present invention, are the same as those described for the iridium complex of the general formula (1).

The iridium complex represented by the general formula (6) can be synthesized by the method described in Organometallics 2006, 25, 2505, or the like. For example, [{ IrH (binap)}₂ (µ-Cl) ₃] Cl can be synthesized by stirring di-µ-chlorotetrakis(cyclooctene)diiridium ([IrCl(coe)₂]₂) and BINAP in toluene, and then adding hydrochloric acid thereto.

The iridium complex represented by the general formula (7) can be synthesized by the method described in Chemistry Letters 1997, 12, 1215, or the like. For example, [IrCl (binap)]₂ can be synthesized by stirring, for example, di-µ-chlorotetrakis(cyclooctene)diiridium ([IrCl (coe)₂]₂) and BINAP in toluene.

Note that the thus obtained iridium complex represented by the formula (6) or (7) may be used in the form of a solution as it is, or may be used after purification.

Examples of iridium compounds which can be used for forming the iridium complex represented by the formula (6) or (7) include
di-µ-chlorotetrakis(cyclooctene)diiridium ([IrCl (coe)₂]₂),
di-µ-bromotetrakis(cyclooctene)diiridium ([IrBr(coe)₂]₂),
di-µ-iodotetrakis(cyclooctene)diiridium ([IrI(coe)₂]₂),
di-µ-chlorobis(1,5-cyclooctadiene)diiridium ([IrCl(cod)]₂),
di-µ-bromobis(1,5-cyclooctadiene)diiridium ([IrBr(cod)]₂),
di-µ-iodobis(1,5-cyclooctadiene)diiridium ([IrI(cod)]₂),
di-µ-chlorobis(bicyclo[2,2,1]hepta-2,5-diene)diiridi um ([IrCI(nbd)]₂),
di-µ-bromobis(bicyclo[2,2,1]hepta-2,5-diene)diiridiu m ([IrBr(nbd)]₂),
di-µ-iodobis(bicyclo[2,2,1]hepta-2,5-diene)diiridium ([IrI(nbd)]₂), and the like.

The iridium complex of the general formula (1*) of the present invention can be prepared by allowing the iridium complex represented by the general formula (6) and the amine (Q) or a salt thereof to react with each other. Examples of the salt of the amine include hydrochloric acid salts, hydrobromic acid salts, acetic acid salts, trifluoroacetic acid salts, and carbonic acid salts. Hydrochloric acid salts and hydrobromic acid salts are more preferable. The amount of the amine (Q) or the salt thereof is preferably 1 to 100 equivalents relative to the iridium atoms of the iridium complex. If the amount is 1 to 10 equivalents, preferable results can be obtained.

In addition, the iridium complex of the general formula (1*) of the present invention can also be prepared by adding the amine (Q) or the salt thereof to the iridium complex represented by the general formula (7), followed by treatment with a hydrogen halide, or a aqueous solution thereof. The amount of the amine (Q) or the salt thereof is preferably 1 to 10 equivalents relative to the iridium atoms of the iridium complex. If the amount is 1 to 5 equivalents, preferable results can be obtained.

The reaction of these is preferably performed in a solvent. Specific examples of the solvent include aromatic hydrocarbon solvents such as toluene and xylene; aliphatic hydrocarbon solvents such as hexane and heptane; halogen-containing hydrocarbon solvents such as methylene chloride; alcohol solvents such as methanol, ethanol, and isopropanol; ether solvents such as diethyl ether, tetrahydrofuran, and 1,4-dioxane; and other organic solvents such as acetonitrile, dimethylformamide, and dimethyl sulfoxide. These solvents can be used alone or as a mixture solvent of two or more kinds.

Examples of the hydrogen halide or the hydrohalic acid include hydrogen halides such as hydrogen chloride, hydrogen bromide, and hydrogen iodide; and hydrohalic acids such as hydrochloric acid, hydrobromic acid, and hydroiodic acid. Hydrohalic acids are preferable from the viewpoint of handling. Each of these hydrogen halides and hydrohalic acids is preferably used in an amount within about 10 equivalents relative to iridium atoms.

When m=1, the general formula (1) represents an iridium complex represented by the following general formula (1a):

IrHZ₂ (PP) (Q) (1a)

wherein Z represents a halogen atom, PP represents a bisphosphine, and Q represents an amine.

Specific examples of the iridium complex represented by the general formula (1a) include
IrHCl₂(dm-segphos)(p-anisidine),
IrHCl₂(binap)(N-Me-p-anisidine), and the like.

Meanwhile, when m=2, the general formula (1) represents an iridium complex represented by the following general formula (1b):

[IrHZ(PP)(Q)₂]Z (Ib)

wherein Z represents a halogen atom, PP represents a bisphosphine, and Q represents an amine.

Specific examples of the iridium complex represented by the general formula (1b) include [IrHCl(dm-segphos)(N-Me-p-anisidine)₂]Cl, [IrHCl(dm-binap)(N-Me-p-anisidine)₂]Cl, and the like.

Note that examples of the halogen atom, the bisphosphine ligand, and the amine in each of the general formula (1a) and (1b) are the same as those described for the iridium complex of the general formula (1).

The thus obtained iridium complex of the general formula (1) of the present invention, in particular, the thus obtained iridium complex having the optically active ligand, is suitably used for a method for producing an optically active compound. Specific reactions for which the iridium complex is used include asymmetric 1,4-addition reactions, asymmetric hydroformylation reactions, asymmetric hydrocyanation reactions, asymmetric hydroamination reactions, asymmetric Heck reactions, and asymmetric hydrogenation reactions. The iridium complex is used particularly advantageously for asymmetric hydrogenation reactions.

Examples of the asymmetric hydrogenation reactions include asymmetric hydrogenation of compounds having a prochiral carbon-carbon double bond, such as prochiral enamines, olefins, and enol ethers; asymmetric hydrogenation of (hetero)aromatic compounds; asymmetric hydrogenation of compounds having a prochiral carbon-oxygen double bond, such as prochiral ketones; and asymmetric hydrogenation of compounds having a prochiral carbon-nitrogen double bond, such as prochiral imines.

Examples of the compounds having a carbon-carbon double bond include an olefin compound represented by the general formula (9): 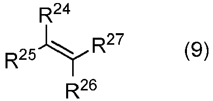 wherein R²⁴, R²⁵, R²⁶, and R²⁷ each represent an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, or an amino group, provided that R²⁴ and R²⁵ are different from each other, and that R²⁶ and R²⁷ are different from each other; and R²⁴ and R²⁶, R²⁴ and R²⁷, or R²⁶ and R²⁷ may be bonded together to form an asymmetric cyclic structure as a whole.

The (hetero)aromatic compound is, for example, a (hetero)aromatic compound represented by the general formula (10): 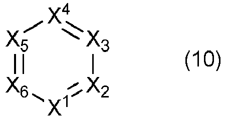 wherein X¹ represents a nitrogen atom or CR²⁸, X² represents a nitrogen atom or CR²⁹, X³ represents a nitrogen atom or CR³⁰, X⁴ represents a nitrogen atom or CR³¹, X⁵ represents a nitrogen atom or CR³², and X⁶ represents a nitrogen atom or CR³³; and R²⁸, R²⁹, R³⁰, R³¹, R³², and R³³ each independently represent a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, a hydroxy group, an amino group, or a halogen atom, provided that cases where all X¹, X², X³, X⁴, X⁵, and X⁶ are nitrogen atoms are excluded.

Another example of the (hetero)aromatic compound is a (hetero)aromatic compound represented by the general formula (11): 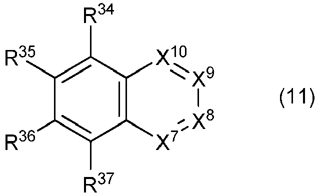 wherein X⁷ represents a nitrogen atom or CR³, X⁸ represents a nitrogen atom or CR³⁹, X⁹ represents a nitrogen atom or CR⁴⁰, and X¹⁰ represents a nitrogen atom or CR⁴¹; and R³⁴, R³⁵, R³⁶, R³⁷, R³⁸, R³⁹, R⁴⁰, and R⁴¹ each independently represent a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, a hydroxy group, an amino group, or a halogen atom, provided that cases where all X⁷, X⁸, X⁹, and X¹⁰ are nitrogen atoms are excluded.

Still another example of the (hetero)aromatic compound is a (hetero)aromatic compound represented by the general formula (12): 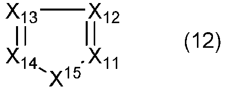 wherein X¹¹ represents a nitrogen atom or CR⁴², X¹² represents a nitrogen atom or CR⁴³, X¹³ represents a nitrogen atom or CR⁴⁴, X¹⁴ represents a nitrogen atom or CR⁴⁵, and X¹⁵ represents an oxygen atom, a sulfur atom, or NR⁴⁶; R⁴², R⁴³ , R⁴⁴, and R⁴⁵ each independently represent a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, a hydroxy group, an amino group, or a halogen atom; and R⁴⁶ represents a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, or a substituted carbamoyl group, provided that cases where all X¹¹, X¹², X¹³, and X¹⁴ are nitrogen atoms are excluded.

Still another example of the (hetero)aromatic compound is a(hetero)aromatic compound represented by the general formula (13): 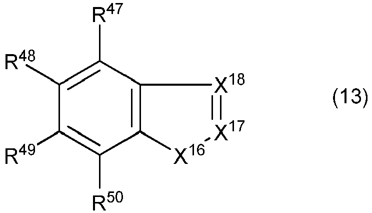 wherein R⁴⁷, R⁴⁸, R⁴⁹, and R⁵⁰ each independently represent a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, a hydroxy group, an amino group, or a halogen atom; X¹⁷ represents a nitrogen atom or CR⁵¹, X¹⁸ represents a nitrogen atom or CR⁵², and X¹⁶ represents an oxygen atom, a sulfur atom, or NR⁵³; R⁵¹ and R⁵² each independently represent a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, a substituted carbamoyl group, a cyano group, an acylamino group, a hydroxy group, an amino group, or a halogen atom; and R⁵³ represents a hydrogen atom, an alkyl group which may have a substituent, a (hetero)aryl group which may have a substituent, an aralkyl group which may have a substituent, an acyl group, a carboxyl group, an alkoxycarbonyl group, a carbamoyl group, or a substituted carbamoyl group, provided that cases where both X¹⁷ and X¹⁸ are nitrogen atoms are excluded.

Examples of the alkyl group represented by any one of R²⁴ to R²⁷, R²⁸ to R³³, R³⁴ to R⁴¹, R⁴² to R⁴⁶, and R⁴⁷ to R⁵³ in the compounds represented by the general formula (9), (10), (11), (12), and (13) include alkyl groups having 1 to 8 carbon atoms such as a methyl group, an ethyl group, a n-propyl group, an isopropyl group, a n-butyl group, an isobutyl group, a sec-butyl group, a tert-butyl group, a n-pentyl group, a tert-pentyl group, and a n-hexyl group. Examples of the substituent which the alkyl group may have include halogen atoms such as a fluorine atom; alkoxy groups having 1 to 6 carbon atoms such as a methoxy group and an ethoxy group; and the like. Examples of the (hetero)aryl group include phenyl, naphthyl, pyridyl, pyrimidinyl, furyl, thienyl, and the like. Examples of the substituent thereof include alkyl groups having 1 to 6 carbon atoms such as a methyl group, an ethyl group, a n-propyl group, an isopropyl group, a n-butyl group, an isobutyl group, a sec-butyl group, a tert-butyl group, a n-pentyl group, a tert-pentyl group, and a n-hexyl group; alkoxy groups having 1 to 6 carbon atoms such as a methoxy group, an ethoxy group, a n-propoxy group, an isopropoxy group, a n-butoxy group, an isobutoxy group, a sec-butoxy group, a tert-butoxy group, a n-pentyloxy group, a tert-pentyloxy group, and a n-hexyloxy group; halogen atoms such as a fluorine atom, a chlorine atom, and a bromine atom; and the like. Examples of the alkyl in the aralkyl group include those having 1 to 12 carbon atoms. Examples of the substituent which the aralkyl group may have include alkyl groups having 1 to 6 carbon atoms such as a methyl group and an ethyl group; alkoxy groups having 1 to 6 carbon atoms such as a methoxy group and ethoxy group; halogen atoms such as a fluorine atom and a chlorine atom; and the like. Examples of the acyl group include an acetyl group, a propanoyl group, a butyryl group, a pivaloyl group, a benzoyl group, and the like. Examples of the alkoxycarbonyl group include a methoxycarbonyl group, an ethoxycarbonyl group, a n-propoxycarbonyl group, an isopropoxycarbonyl group, a tert-butoxycarbonyl group, a benzyloxycarbonyl group, and the like. Examples of the substituted carbamoyl group include a dimethylcarbamoyl group, a diethylcarbamoyl group, a dibenzylcarbamoyl group, and the like. Examples of the acylamino group include an acetylamino group, a tert-butoxycarbonylamino group, a benzyloxycarbonylamino group, and the like. When R²⁴ and R²⁶, R²⁴ and R²⁷, or R²⁶ and R²⁷ are bonded together to form a disymmetric cyclic structure as a whole, the structure is preferably a 5- membered or 6-membered ring structure.

Among the organic compounds having multiple bonds, an example of the compound having a carbon-oxygen double bond is a ketone compound represented by the general formula (14): 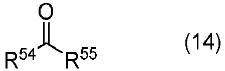 wherein R⁵⁴ and R⁵⁵ are different from each other, and each represent an alkyl group which may have a substituent, a (hetero)aryl group which may have a substituent, or an aralkyl group which may have a substituent; and R⁵⁴ and R⁵⁵ may be bonded together to form a disymmetric cyclic ketone as a whole. An example of the compound having a carbon-nitrogen double bond is an imine compound represented by the general formula (15) : 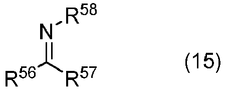 wherein R⁵⁶ and R⁵⁷ are different from each other, and each represent an alkyl group which may have a substituent, a (hetero)aryl group which may have a substituent, or an aralkyl group which may have a substituent; R⁵⁸ represents a hydrogen atom, an alkyl group which may have a substituent, a (hetero) aryl group which may have a substituent, or an aralkyl group which may have a substituent; and R⁵⁶ and R⁵⁷, R⁵⁶ and R⁵⁸, or R⁵⁷ and R⁵⁸ may be bonded together to form a disymmetric cyclic imine as a whole.

Examples of the alkyl group represented by any one of R⁵⁴ and R⁵⁵ of the compound represented by the general formula (14) and R⁵⁶, R⁵⁷, and R⁵⁸ of the compound represented by the general formula (15) include alkyl groups having 1 to 8 carbon atoms.
Examples of the substituent which the alkyl group may have include halogen atoms such as such as a fluorine atom and a chlorine atom; alkoxy groups having 1 to 6 carbon atoms such as a methoxy group and an ethoxy group; and the like. Examples of the (hetero) aryl group include phenyl, naphthyl, pyridyl, pyrimidinyl, furyl, thienyl, and the like. Examples of the substituent include alkyl groups having 1 to 6 carbon atoms, alkoxy groups having 1 to 6 carbon atoms, halogen atoms, and the like. Examples of the alkyl of the aralkyl group include those having 1 to 12 carbon atoms. Examples of the substituent which the aralkyl group may have include alkyl groups having 1 to 6 carbon atoms such as a methyl group and an ethyl group; alkoxy groups having 1 to 6 carbon atoms such as a methoxy group and an ethoxy group; halogen atoms such as a fluorine atom and a chlorine atom; and the like.

Examples of the cyclic ketone which may be substituted represented by the general formula (14) in a case where R⁵⁴ and R⁵⁵ are bonded together to form a disymmetric cyclic ketone as a whole include compounds having a cycloalkenone structure having 1 to 8 carbon atoms, compounds having a 1-indanone structure, compounds having a 2-indanone skeleton, compounds having a 1-tetralone structure, compounds having a 2-tetralone structure, compounds having a 1-benzosuberone structure, and the like. Examples of the substituent include alkyl groups having 1 to 6 carbon atoms, alkoxy groups having 1 to 6 carbon atoms, halogen atoms, aryl groups, and the like.

Examples of the cyclic imine which may be substituted represented by the general formula (15) in a case where R⁵⁶ and R⁵⁷, R⁵⁶ and R⁵⁸, or R⁵⁷ and R⁵⁸ are bonded together to form a disymmetric cyclic imine as a whole include compounds having a 3,4-dihydro-2H-pyrrole skeleton, compounds having a 2,3,4,5-tetrahydropyridine skeleton, compounds having a 3H-indole skeleton, compounds having a 3,4-dihydroquinoline skeleton, compounds having a 3,4-dihydro isoquinoline skeleton, and the like. Examples of the substituent include alkyl groups having 1 to 6 carbon atoms, alkoxy groups having 1 to 6 carbon atoms, halogen atoms, aryl groups, and the like.

specific examples of the compounds represented by the general formula (10) to (15) include acetophenone, propiophenone, butyrophenone, isobutyrophenone, chloromethyl phenyl ketone, bromomethyl phenyl ketone, 2-acetylpyridine, 3-acetylpyridine, (o-methoxy)acetophenone, (o-ethoxy)acetophenone, (o-propoxy)acetophenone, (o-benzyloxy)acetophenone, α-acetonaphthone, p-chlorophenyl methyl ketone, p-bromophenyl methyl ketone, p-cyanophenyl methyl ketone, phenyl benzyl ketone, phenyl (o-tolylmethyl) ketone, phenyl (m-tolylmethyl) ketone, phenyl (p-tolylmethyl) ketone, 2-butanone, 2-pentanone, 2-hexanone, 2-heptanone, 2-octanone, 2-nonanone, 2-decanone, cyclohexyl methyl ketone, cyclohexyl ethyl ketone, cyclohexyl benzyl ketone, t-butyl methyl ketone, 3-quinuclidinone, 1-indanone, 2-indanone, 1-tetralone, 2-tetralone, benzyl (2-pyridyl) ketone, benzyl (3-pyridyl) ketone, benzyl (2-thiazolyl) ketone, 2-methylquinoline, 2-phenylquinoline, 2-phenylquinoxaline, 2,6-dimethylquinoline, 3,4-dihydro-5-phenyl-2H-pyrrole, 2,3,4,5-tetrahydro-6-phenylpyridine, 1-methyl-3,4-dihydroisoquinoline, 6,7-dimethoxy-1-methyl-3,4-dihydroisoquinoline, 1-phenyl-3,4-dihydroisoquinoline, 1-methyl-3,4-dihydro-9H-pyrido[3,4-b]indole, α-methylbenzylidenebenzylamine, 2-methylbenzofuran, 2-isopropylbenzofuran, 2-phenylbenzothiophene, and the like.

The iridium complex represented by the general formula (1) of the present invention is useful as a catalyst for reduction of multiple bonds in organic compounds, in particular, for reduction of compounds having a carbon-carbon double bond, aromatic compounds, and compounds having a carbon-hetero atom double bond. Moreover, when an optically active isomer is employed as the ligand bisphosphine, the iridium complex is useful also as a catalyst for asymmetric hydrogenation reaction. When the iridium complex represented by the general formula (1) of the present invention is used as a catalyst, the complex may be used after the purity of the complex is increased by means of, for example, concentration, vacuum concentration, solvent extraction, washing, recrystallization, or the like after the reaction for synthesis of the iridium complex. Alternatively, the complex may be used as a catalyst for reduction reaction without purification of the complex.

Moreover, instead of the synthesizing of the iridium complex in advance as described above, the asymmetric hydrogenation can be conducted by adding the iridium complex represented by the general formula (6) or (7), the amine or the salt thereof, and an asymmetric hydrogenation substrate to a reaction system (in situ method).

The asymmetric hydrogenation is carried out as follows. Specifically, a substrate to be hydrogenated is dissolved in a solvent which does not inhibit the asymmetric hydrogenation reaction, for example, in an alcohol solvent such as methanol, ethanol, or isopropanol, tetrahydrofuran, diethyl ether, methylene chloride, acetone, ethyl acetate, benzene, toluene, N,N-dimethylformamide, acetonitrile, or a mixture solvent thereof. The catalyst is added in an amount of 1/10 to 1/10,000 molar equivalents, and preferably approximately 1/50 to 1/3,000 molar equivalents relative to the substrate. Then, the hydrogenation is carried out at a hydrogen pressure of approximately 1 to 10 MPa, preferably approximately 3 to 7 MPa, and at a reaction temperature of approximately -20 to 100 °C, preferably approximately 20 to 80°C, for approximately 5 to 30 hours, preferably for approximately 10 to 20 hours.

### [Examples]

The present invention will be described in detail by showing examples below. However, the present invention is not limited to these examples at all. Note that the following analytical instruments were used in the examples.
Nuclear magnetic resonance spectra (NMR): MERCURY300-C/H (VARIAN)
Melting point (mp): MP-500D (Yanako)
Infrared absorption spectra (IR): FT/IR-230 (JASCO Corp.)
Gas chromatography (GLC): GC-14A (Shimadzu Corp.)

### (Example 1)

### Synthesis of IrHCl₂((R)-dm-segphos)(p-anisidine)

Into a nitrogen-purged Schlenk tube, [{IrH((R)dm-segphos)}₂(µ-Cl)₃]Cl (50mg, 0.051 mmol), p-anisidine (6.2 mg, 0.05 mmol), and methylene chloride (2 ml) were added. After stirring at room temperature for one hour, the reaction mixture was concentrated to obtain the title compound (44 mg, yield: 80%).
¹H NMR (CD₂Cl₂) : δ 7.7-6.55 (m, 12H), 7.25 (dd, J = 8.4, 11.5 Hz, 1H), 6.65 (dd, J = 1.1, 8.4 Hz, 1H), 6.50 (d, J = 8.9 Hz, 2H), 6.37 (d, J = 8.9 Hz, 2H), 6.21 (dd, J = 1.5, 8.4 Hz, 1H), 6.01 (dd, J = 8.4, 11.8 Hz, 1H), 6.00-5.90 (m, 1H), 5.88 (d, J = 1.1 Hz, 1H), 5.75 (d, J = 1.1 Hz, 1H), 5.67 (d, J = 1.1 Hz, 1H), 5.60 (d, J = 1.1 Hz, 1H), 4.50-4.30 (m, 1H), 3.67 (s, 3H), 2.30 (s, 6H), 2.29 (s, 6H), 2.24 (s, 6H), 2.14 (brs, 3H), 2.05 (brs, 3H), -20.41 (dd, J = 13.9, 21.4 Hz, 1H).
³¹P NMR (CD₂Cl₂) : δ -0.17 (br), -6.79 (br).
HRMS (ESI) : m/z calcd for C₅₃H₅₃NO₅P₂ClIr [M-Cl]⁺ 1074.2789; m/z found 1074.2778.

### (Example 2)

### Synthesis of IrHCl₂((R)-binap)(N-Me-p-anisidine)

Into a nitrogen-purged Schlenk tube, [IrCl (coe)₂]₂ (200 mg, 0.45 mmol), (R)-BINAP (308 mg, 0.49 mmol), and toluene (10 ml) were added. After stirring at room temperature for one hour, N-methyl-p-anisidine (102 mg, 0.74 mmol) was added, followed by stirring at the same temperature for 30 minutes. Then, concentrated hydrochloric acid (160 µl, 2.10 mmol) was added. After stirring for 4 hours, the precipitates were filtrated to obtain the light yellow complex (270 mg, yield: 59.6%).
¹H NMR (C₆D₆) : δ 8.70-6.20 (m, 36H), 3.12 (s, 3H), 2.82 (s, 3H), -20.26 (dd, J = 14.1, 19.5 Hz, 1H);
³¹P NMR (C₆D₆) : δ-0.18 (m), -3.57 (m).

### (Example 3)

### synthesis of IrHCl₂((R)-binap)(N-Me-p-anisidine)

Into a nitrogen-purged Schlenk tube, [{IrH((R)-binap)}₂(µ-Cl)₃]Cl (50 mg, 0.056 mmol), N-methyl-p-anisidine hydrochloride (24 mg, 0.138 mmol), and toluene (5 ml) were added. After stirring at room temperature for one hour, the precipitates were filtrated to obtaine the light yellow complex (57 mg, yield: 99.2%).

### (Example 4)

### synthesis of [IrHCl((R)-dm-segphos)(N-Me-p-anisidine)₂]Cl

Into a nitrogen-purged Schlenk tube, [{IrH((R)-dm-segphos)}₂(µ-Cl)₃]Cl (72.8 mg, 0.037 mmol), N-methyl-p-anisidine hydrochloride (64.1 mg, 0.37 mmol), and THF (3 ml) were added. After stirring at room temperature for one hour, the precipitates were filtered to obtain the title complex (88 mg, yield: 95%).
¹H NMR (C₆D₆): δ 8.18 (s, 1H), 8.15 (s, 1H), 8.10-7.40 (m, 4H), 7.74 (d, J = 8.4 Hz, 4H), 7.61 (dd, J = 8.4, 11.6 Hz, 1H), 7.65-7.50 (m, 2H), 6.82 (s, 1H), 6.78 (s, 1H), 6.75 (s, 1H), 6.70-6.60 (m, 1H), 6.66 (s, 1H), 6.54 (d, J = 8.4 Hz, 4H), 6.38 (d, J = 8.4 Hz, 1H), 6.02 (d, J = 8.2 Hz, 1H), 5.41 (s, 1H), 5.29 (s, 1H), 5.27 (s, 1H), 5.21 (s, 1H), 3.16 (s, 6H), 2.90 (s, 6H), 2.20-1.95 (m, 24H), -20.5 (dd, J = 14.5, 19.2 Hz, 1H).
³¹P NMR (C₆D₆) : δ0.17 (m), -4.37 (m).

### (Example 5)

### Synthesis of [IrHCl((R)-dm-binap)(N-Me-p-anisidine)₂]Cl

Into a nitrogen-purged Schlenk tube, [{IrH((R)-dm-binap)}₂(µ-Cl)₃]Cl (200 mg, 0.100 mmol), N-methyl-p-anisidine hydrochlorde (173.6 mg, 1.00 mmol), and THF (10 ml) were added. After stirring at room temperature for one hour, the precipitates were filtrered to obtain the title complex (233 mg, yield: 92%).
¹H NMR (C₆D₆) : δ 8.55-6.55 (m, 26H), 6.47 (d, J = 9.0 Hz, 4H), 6.20-6.05 (m, 2H), 3.12 (s, 6H), 2.85 (s, 6H), 2.20 (s, 6H), 2.12 (s, 6H), 1.78 (s, 6H), 1.68 (s, 6H), -20.84 (dd, J = 15.0, 19.2 Hz, 1H).
³¹P NMR (C₆D₆) : δ -0.89 (m), -6.51 (m).

### (Example 6)

### Asymmetric Hydrogenation Reaction of 2-Methylquinoline

Into a 100-ml stainless steel autoclave, IrHCl₂((R)-binap)(N-Me-p-anisidine) (21.6 mg, 0.021 mmol) was added. After nitrogen purge, methylene chloride (5.0 ml) and 2-methylquinoline (60.1 mg, 0.420 mmol) were added. Subsequently, hydrogen was introduced at a pressure of 5.0 MPa. followed by stirring at 80°C for 18 hours. After cooling, the reaction product was analyzed by GC. As a result, the conversion was 96%, and the enantiomeric excess was 49% ee.

### (Example 7)

### Asymmetric Hydrogenation Reaction of 2-Methylquinoline (in situ method)

Into a 100-ml stainless steel autoclave, [{IrH((R)-binap)}₂(µ-Cl)₃]Cl (18.4 mg, 0.010 mmol) and N-methyl-p-anisidine (28.6 mg, 0.208 mmol) were added. After nitrogen purge, methylene chloride (5.0 ml) and 2-methylquinoline (60.6 mg, 0.423 mmol) were added. Subsequently, hydrogen was introduced at a pressure of 5.0 MPa, followed by stirring at 80°C for 18 hours. After cooling, the reaction product was analyzed by GC. As a result, the conversion was 95%, and the enantiomeric excess was 56% ee.

### (Example 8)

### Asymmetric Hydrogenation Reaction of 2-Methylquinoline (in situ method)

Into a 100-ml stainless steel autoclave, [{IrH((R)-binap)}₂(µ-Cl)₃]Cl (18.6 mg, 0.010 mmol) and N-methyl-p-anisidine hydrochloride (36.5 mg, 0.210 mmol) were added. After nitrogen purge, methylene chloride (5.0 ml) and 2-methylquinoline (60.1 mg, 0.420 mmol) were added. Subsequently, hydrogen was introduced at a pressure of 5.0 MPa, followed by stirring at 80°C for 18 hours. After cooling, the reaction product was analyzed by GC. As a result, the conversion was 96%, and the enantiomeric excess was 65% ee.

### (Comparative Example 1)

### Asymmetric Hydrogenation Reaction of 2-Methylquinoline (in the absence of amine)

Into a 100-ml stainless steel autoclave, [{IrH((S)-binap)}₂(µ-Cl)₃]Cl (18.6 mg, 0.010 mmol) was added. After nitrogen purge, methylene chloride (5.0 ml) and 2-methylquinoline (60.9 mg, 0.425 mmol) were added. Subsequently, hydrogen was introduced at a pressure of 5.0 MPa, followed by stirring at 80°C for 18 hours. After cooling, the reaction product was analyzed by GC. As a result, the conversion was 96%, and the enantiomeric excess was 38% ee.

### (Example 9)

### Asymmetric Hydrogenation Reaction of 2-Methylquinoline

Into a 100-ml stainless steel autoclave, [IrHCl((R)-dm-segphos)(N-Me-p-anisidine)₂]Cl (11.8 mg, 0.0094 mmol) was added. After nitrogen purge, methylene chloride (2.5 ml) and 2-methylquinoline (30.1 mg, 0.210 mmol) were added. Subsequently, hydrogen was introduced at a pressure of 5.0 MPa, followed by stirring at 80°C for 18 hours. After cooling, the reaction product was analyzed by GC. As a result, the conversion was 92%, and the enantiomeric excess was 64% ee.

### (Example 10)

### Asymmetric Hydrogenation Reaction of 2-Methylquinoline (in situ method)

The same operations as those in Example 7 were carried out, except that [{IrH((R)-dm-segphos)}₂(µ-Cl)₃]Cl was used instead of [{IrH((R)-binap)}₂(µ-Cl)₃]Cl. As a result, the conversion was 92%, and the enantiomeric excess was 72% ee.

### (Example 11)

### Asymmetric Hydrogenation Reaction of 2-Methylquinoline (in situ method)

The same operations as those in Example 10 were carried out, except that the solvent was changed from methylene chloride to tetrahydrofuran. As a result, the conversion was 99%, and the enantiomeric excess was 75%ee.

## Claims

1. An iridium complex of the following general formula (1) :
IrHZ₂(PP)(Q)ₘ (1)
wherein Z represents a halogen atom selected from the group consisting of a chlorine atom, a bromine atom and a iodine atom, PP represents a bisphosphine, Q represents an amine, and m represents 1 or 2, wherein PP in the general formula (1) is an optically active bisphosphine,
wherein
Q in the general formula (1) is an amine represented by the following general formula (3): 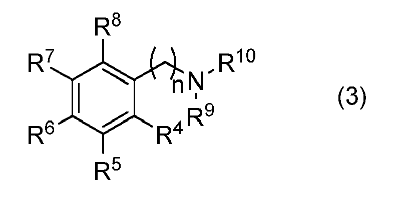 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1, and
wherein
the optically active bisphosphine of the general formula (1) is an optically active bisphosphine represented by the following formula (4) or (5): 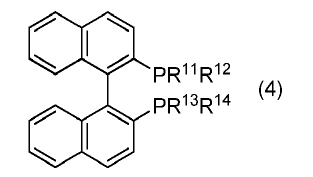 wherein R¹¹, R¹², R¹³, and R¹⁴ each independently represent a phenyl group which may have a substituent selected from alkyl groups and alkoxy groups, 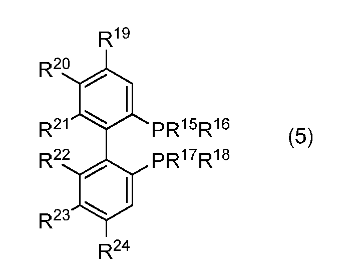 wherein R¹⁵, R¹⁶, R¹⁷, and R¹⁸ each independently represent a phenyl group which may have a substituent selected from alkyl groups and alkoxy groups; R¹⁹, R²⁰, R²¹, R²², R²³, and R²⁴ may be the same or different, and each represent a hydrogen atom, an alkyl group, an alkoxy group, an acyloxy group, a halogen atom, a haloalkyl group, or a dialkylamino group; R²⁰ and R²¹, as well as R²² and R²³, may form a methylene chain which may have a substituent or a (poly)methylenedioxy group which may have a substituent; and R²¹ and R²² may form a methylene chain which may have a substituent or a (poly)methylenedioxy group which may have a substituent, provided that neither R²¹ and R²² are a hydrogen atom.

2. A method for producing an iridium complex of the following general formula (1*) :
IrHZ₂(PP*)(Q)ₘ (1*)
wherein Z represents a halogen atom, PP* represents an optically active bisphosphine, Q represents an amine, and m represents 1 or 2,
the method comprising allowing one or more equivalents of an amine or a salt thereof to react with an iridium complex represented by the following general formula (6):
[{IrH(PP*)}₂(µ-Z)₃]Z (6)
wherein Z represents a halogen atom, and PP* represents an optically active bisphosphine.

3. A method for producing an iridium complex of the following general formula (1*):
IrHZ₂(PP*)(Q)ₘ (1*)
wherein Z represents a halogen atom, PP* represents an optically active bisphosphine, Q represents an amine, and m represents 1 or 2,
the method comprising allowing one or more equivalents of an amine or a salt thereof to react with an iridium complex represented by the following general formula (7), and subsequently allowing one or more equivalents of a hydrogen halide HZ (where Z represents a halogen atom) or an aqueous solution thereof to react therewith:
[IrZ(PP*)]₂ (7)
wherein Z represents a halogen atom, and PP* represents an optically active bisphosphine.

4. The production method according to claim 2 or 3, wherein
the amine is an amine represented by the following general formula (2):
NR¹R²R³ (2)
wherein R¹, R² and R³ each independently represent a hydrogen atom, an alkyl group having 1 to 6 carbon atoms, or an aryl group which may have a substituent.

5. The production method according to claim 2 or 3, wherein
the amine is an amine represented by the following general formula (3): 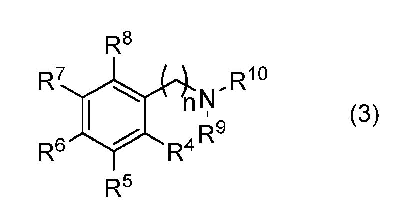 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1.

6. An asymmetric hydrogenation catalyst comprising the iridium complex according to claim 1.

7. A method for producing an optically active compound, comprising performing an asymmetric hydrogenation of a compound having a prochiral carbon-carbon double bond, a prochiral carbon-oxygen double bond and/or a prochiral carbon-nitrogen double bond, or a (hetero)aromatic compound, wherein
the asymmetric hydrogenation is performed in the presence of an iridium complex represented by the following general formula (6):
[{IrH(PP*)}₂(µ-Z)₃]Z (6)
wherein Z represents a halogen atom and PP* represents an optically active bisphosphine, and in the presence of an amine represented by the following general formula (3) or a salt thereof: 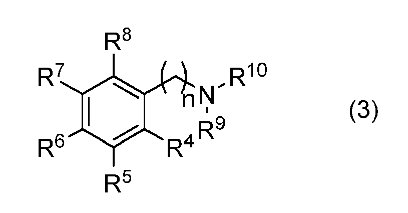 wherein R⁴, R⁵, R⁶, R⁷, and R⁸ each independently represent a hydrogen atom, an alkyl group having 1 to 5 carbon atoms, an alkoxy group having 1 to 5 carbon atoms, a halogen atom, a nitro group, or a cyano group; R⁹ and R¹⁰ each independently represent a hydrogen atom, an alkyl group having 1 to 5 carbon atoms, or an aryl group which may have a substituent; and n represents 0 or 1.

## Patentansprüche

1. Iridiumkomplex mit der folgenden allgemeinen Formel (1):
IrHZ₂(PP)(Q)ₘ (1)
wobei Z ein Halogenatom ist, ausgewählt aus der Gruppe bestehend aus einem Chloratom, einem Bromatom und einem Iodatom, PP ein Bisphosphin darstellt, Q ein Amin darstellt, und m 1 oder 2 bedeutet, wobei PP in der allgemeinen Formel (1) ein optisch aktives Bisphosphin ist,
wobei
Q in der allgemeinen Formel (1) ein Amin ist, das durch die allgemeine Formel (3) dargestellt wird: 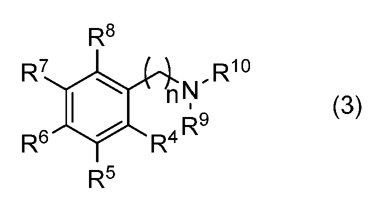 wobei R⁴, R⁵, R⁶, R⁷ und R⁸ jeweils unabhängig ein Wasserstoffatom, eine Alkylgruppe mit 1 bis 5 Kohlenstoffatomen, eine Alkoxygruppe mit 1 bis 5 Kohlenstoffatomen, ein Halogenatom, eine Nitrogruppe oder eine Cyanogruppe darstellen; R⁹ und R¹⁰ stellen jeweils unabhängig ein Wasserstoffatom, eine Alkylgruppe mit 1 bis 5 Kohlenstoffatomen oder eine Arylgruppe, die einen Substituenten aufweisen kann, dar; und n bedeutet 0 oder 1, und wobei
das optisch aktive Bisphosphin der allgemeinen Formel (1) ein optisch aktives Bisphosphin ist, das durch die folgende Formel (4) oder (5) dargestellt wird: 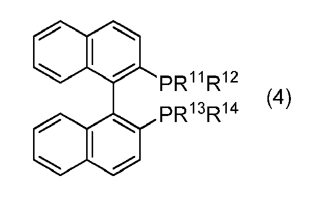 wobei R¹¹, R¹², R¹³ und R¹⁴ jeweils unabhängig eine Phenylgruppe darstellen, die einen Substituenten ausgewählt aus Alkylgruppen und Alkoxygruppen aufweisen
kann, 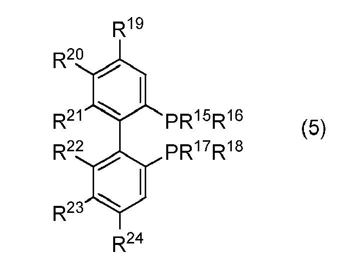 wobei R¹⁵, R¹⁶, R¹⁷ und R¹⁸ jeweils unabhängig eine Phenylgruppe darstellen, die einen Substituenten ausgewählt aus Alkylgruppen und Alkoxygruppen aufweisen kann; R¹⁹, R²⁰, R²¹, R²², R²³ und R²⁴ können gleich oder verschieden sein, und jeder stellt ein Wasserstoffatom, eine Alkylgruppe, eine Alkoxygruppe, eine Acyloxygruppe, ein Halogenatom, eine Haloalkylgruppe oder eine Dialkylaminogruppe dar; R²⁰ und R²¹ sowie R²² und R²³ können eine Methylenkette bilden, welche einen Substituenten aufweisen kann, oder eine (Poly)Methylendioxygruppe, welche einen Substituenten aufweisen kann; und R²¹ und R²² können eine Methylenkette bilden, welche einen Substituenten aufweisen kann, oder eine (Poly)Methylendioxygruppe, welche einen Substituenten aufweisen kann, mit der Maßgabe, dass weder R²¹ noch R²² ein Wasserstoffatom sind.

2. Verfahren zur Herstellung eines Iridiumkomplexes mit der folgenden allgemeinen Formel (1*):
IrHZ₂(PP*)(Q)ₘ (1*)
wobei Z ein Halogenatom darstellt, PP* ein optisch aktives Bisphosphin darstellt, Q ein Amin darstellt, und m 1 oder 2 bedeutet,
wobei das Verfahren umfasst, dass es ermöglicht wird, dass ein oder mehrere Äquivalente eines Amins oder eines Salzes davon mit einem Iridiumkomplex, dargestellt durch die folgende allgemeine Formel (6), reagieren:
[{IrH(PP*)}₂(µ-Z)₃]Z (6)
wobei Z ein Halogenatom darstellt und PP* ein optisch aktives Bisphospin darstellt.

3. Verfahren zur Herstellung eines Iridiumkomplexes der folgenden allgemeinen Formel (1*):
IrHZ₂(PP*)(Q)ₘ (1*)
wobei Z ein Halogenatom darstellt, PP* ein optisch aktives Bisphospin darstellt, Q ein Amin darstellt und m 1 oder 2 bedeutet,
wobei das Verfahren umfasst, dass es ermöglicht wird, dass ein oder mehrere Äquivalente eines Amins oder eines Salzes davon mit einem Iridiumkomplex, dargestellt durch die folgende allgemeine Formel (7), reagieren, und es anschließend ermöglicht wird, dass ein oder mehrere Äquivalente eines Wasserstoffhalids HZ (bei dem Z ein Halogenatom darstellt) oder einer wässrigen Lösung davon damit reagiert:
[IrZ(PP*)]₂ (7)
wobei Z ein Halogenatom darstellt und PP* ein optisch aktives Bisphospin darstellt.

4. Herstellungsverfahren gemäß Patentanspruch 2 oder 3, wobei
das Amin ein Amin dargestellt durch die folgende allgemeine Formel (2) ist:
NR¹R²R³ (2)
wobei R¹, R² und R³ jeweils unabhängig ein Wasserstoffatom, eine Alkylgruppe mit 1 bis 6 Kohlenstoffatomen, oder eine Arylgruppe, die einen Substituenten aufweisen kann, darstellen.

5. Herstellungsverfahren gemäß Patentanspruch 2 oder 3, wobei
das Amin ein Amin ein Amin dargestellt durch die folgende allgemeine Formel (3) ist: 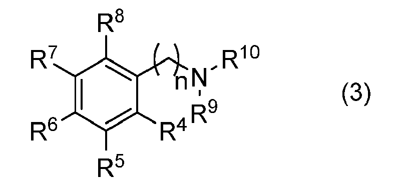 wobei R⁴, R⁵, R⁶, R⁷ und R⁸ jeweils unabhängig ein Wasserstoffatom, eine Alkylgruppe mit 1 bis 5 Kohlenstoffatomen, ein Halogenatom, eine Nitrogruppe oder eine Cyanogruppe darstellen; R⁹ und R¹⁰ jeweils unabhängig ein Wasserstoffatom, eine Alkylgruppe mit 1 bis 5 Kohlenstoffatomen, oder eine Arylgruppe, die einen Substituenten aufweisen kann, darstellen; und n 0 oder 1 bedeutet.

6. Asymmetrischer Hydrierungskatalysator enthaltend den Iridiumkomplex gemäß des Patentanspruchs 1.

7. Verfahren zur Herstellung einer optisch aktiven Verbindung, umfassend die Durchführung einer asymmetrischen Hydrierung einer Verbindung mit einer prochiralen Kohlenstoff-Kohlenstoff-Doppelbindung, einer prochiralen Kohlenstoff-Sauerstoff-Doppelbindung und/oder einer prochiralen Kohlenstoff-Stickstoff-Doppelbindung oder einer (Hetero)aromatischen Verbindung, wobei
die asymmetrische Hydrierung in Gegenwart eines Iridiumkomplexes dargestellt durch die folgende allgemeine Formel (6) durchgeführt wird:
[{IrH(PP*)}₂(µ-Z)₃]Z (6)
wobei Z ein Halogenatom und PP* ein optisch aktives Bisphosphin darstellt, und in Gegenwart eines Amins dargestellt durch die folgende allgemeine Formel (3) oder eines Salzes davon: 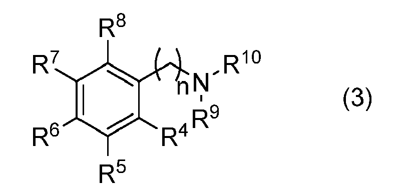 wobei R⁴, R⁵, R⁶, R⁷ und R⁸ jeweils unabhängig ein Wasserstoffatom, eine Alkylgruppe mit 1 bis 5 Kohlenstoffatomen, eine Alkoxygruppe mit 1 bis 5 Kohlenstoffatomen, ein Halogenatom, eine Nitrogruppe oder eine Cyanogruppe darstellen; R⁹ und R¹⁰ jeweils unabhängig ein Wasserstoffatom, eine Alkylgruppe mit 1 bis 5 Kohlenstoffatomen oder eine Arylgruppe, die einen Substituenten aufweisen kann, darstellen; und n 0 oder 1 bedeutet.

## Revendications

1. Complexe d'iridium de la formule générale (1) suivante :
IrHZ₂(PP)(Q)ₘ (1)
où Z représente un atome d'halogène sélectionné dans le groupe constitué par un atome de chlore, un atome de brome et un atome d'iode, PP représente un bisphosphine, Q représente une amine, et m représente 1 ou 2, sachant que PP dans la formule générale (1) est un bisphosphine optiquement actif, sachant que
Q dans la formule générale (1) est une amine représentée par la formule générale (3) suivante : 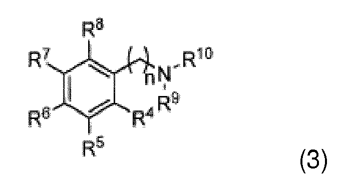 où R⁴, R⁵, R⁶, R⁷, et R⁸ représentent chacun indépendamment un atome d'hydrogène, un groupe alkyle comprenant 1 à 5 atomes de carbone, un groupe alcoxy comprenant 1 à 5 atomes de carbone, un atome d'halogène, un groupe nitro, ou un groupe cyano ; R⁹ et R¹⁰ représentent chacun indépendamment un atome d'hydrogène, un groupe alkyle comprenant 1 à 5 atomes de carbone, ou un groupe aryle pouvant avoir un substituant ; et n représente 0 ou 1, et sachant que
le bisphosphine optiquement actif de la formule générale (1) est un bisphosphine optiquement actif représenté par la formule (4) ou (5) suivante : 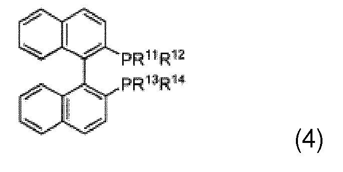 où R¹¹, R¹², R¹³, et R¹⁴ représentent chacun indépendamment un groupe phényle pouvant avoir un substituant sélectionné parmi des groupes alkyles et des groupes alcoxy, 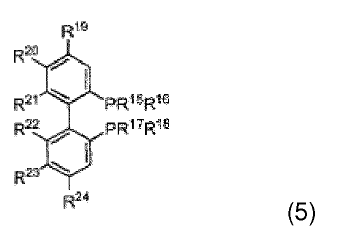 où R¹⁵, R¹⁶, R¹⁷, et R¹⁸ représentent chacun indépendamment un groupe phényle pouvant avoir un substituant sélectionné parmi des groupes alkyles et des groupes alcoxy ; R¹⁹, R²⁰, R²¹, R²², R²³, et R²⁴ peuvent être les mêmes ou différents, et chacun représente un atome d'hydrogène, un groupe alkyle, un groupe alcoxy, un groupe acyloxy, un atome d'halogène, un groupe haloalkyle, ou un groupe dialkylamino ; R²⁰ et R²¹, ainsi que R²² et R²³, peuvent former une chaîne de méthylène pouvant avoir un substituant ou un groupe (poly)méthylènedioxy pouvant avoir un substituant ; et R²¹ et R²² peuvent former une chaîne de méthylène pouvant avoir un substituant ou un groupe (poly)méthylènedioxy pouvant avoir un substituant, à condition que ni R²¹ ni R²² ne soit un atome d'hydrogène.

2. Procédé de production d'un complexe d'iridium de la formule générale (1*) suivante :
IrHZ₂(PP*)(Q)ₘ (1*)
où Z représente un atome d'halogène, PP* représente un bisphosphine optiquement actif, Q représente une amine, et m représente 1 ou 2,
le procédé comprenant le fait de permettre à un ou plusieurs équivalents d'une amine ou d'un sel de celle-ci de réagir avec un complexe d'iridium représenté par la formule générale (6) suivante :
[{IrH(PP*)}₂(µ-Z)₃]Z (6)
où Z représente un atome d'halogène, et PP* représente un bisphosphine optiquement actif.

3. Procédé de production d'un complexe d'iridium de la formule générale (1*) suivante :
IrHZ₂(PP*)(Q)ₘ (1*)
où Z représente un atome d'halogène, PP* représente un bisphosphine optiquement actif, Q représente un amine, et m représente 1 ou 2,
le procédé comprenant le fait de permettre à un ou plusieurs équivalents d'une amine ou d'un sel de celle-ci de réagir avec un complexe d'iridium représenté par la formule générale (7) suivante, et subséquemment de permettre à un ou plusieurs équivalents d'un halogénure d'hydrogène HZ (où Z représente un atome d'halogène) ou d'une solution aqueuse de celui-ci de réagir avec lui :
[IrZ(PP*)]₂ (7)
où Z représente un atome d'halogène, et PP* représente un bisphosphine optiquement actif.

4. Le procédé de production selon la revendication 2 ou 3, dans lequel
l'amine est une amine représentée par la formule générale (2) suivante :
NR¹R¹R³ (2)
où R¹, R² et R³ représentent chacun indépendamment un atome d'hydrogène, un groupe alkyle comprenant 1 à 6 atomes de carbone, ou un groupe aryle pouvant avoir un substituant.

5. Le procédé de production selon la revendication 2 ou 3, dans lequel
l'amine est une amine représentée par la formule générale (3) suivante : 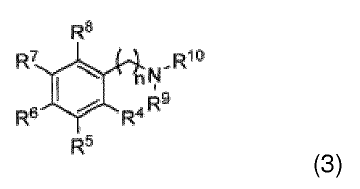 où R⁴, R⁵, R⁶, R⁷, et R⁸ représentent chacun indépendamment un atome d'hydrogène, un groupe alkyle comprenant 1 à 5 atomes de carbone, un groupe alcoxy comprenant 1 à 5 atomes de carbone, un atome d'halogène, un groupe nitro, ou un groupe cyano ; R⁹ et R¹⁰ représentent chacun indépendamment un atome d'hydrogène, un groupe alkyle comprenant 1 à 5 atomes de carbone, ou un groupe aryle pouvant avoir un substituant ; et n représente 0 ou 1.

6. Catalyseur d'hydrogénation asymétrique comprenant le complexe d'iridium selon la revendication 1.

7. Procédé de production d'un composé optiquement actif, comprenant l'exécution d'une hydrogénation asymétrique d'un composé ayant une liaison double carbone-carbone prochirale, une liaison double carbone-oxygène prochirale et/ou une liaison double carbone-azote prochirale, ou un composé (hétéro)aromatique, sachant que
l'hydrogénation asymétrique est exécutée en présence d'un complexe d'iridium représenté par la formule générale (6) suivante :
[{IrH(PP*)}₂(µ-Z)₃]Z (6)
où Z représente un atome d'halogène et PP* représente un bisphosphine optiquement actif, et en présence d'une amine représentée par la formule générale (3) suivante ou d'un sel de celle-ci : 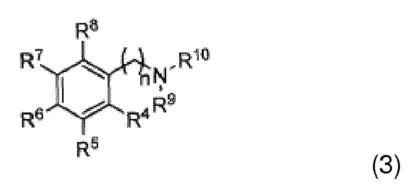 où R⁴, R⁵, R⁶, R⁷, et R⁸ représentent chacun indépendamment un atome d'hydrogène, un groupe alkyle comprenant 1 à 5 atomes de carbone, un groupe alcoxy comprenant 1 à 5 atomes de carbone, un atome d'halogène, un groupe nitro, ou un groupe cyano ; R⁹ et R¹⁰ représentent chacun indépendamment un atome d'hydrogène, un groupe alkyle comprenant 1 à 5 atomes de carbone, ou un groupe aryle pouvant avoir un substituant ; et n représente 0 ou 1.
